# EUROPÄISCHE PATENTSCHRIFT

(11) **EP 2 433 562 B1**
(45) Veröffentlichungstag und Bekanntmachung des Hinweises auf die Patenterteilung: **09.07.2014**
(21) Anmeldenummer: 11179131.5
(22) Anmeldetag: 29.08.2011
(51) Int. Cl.: A61B 5/05

(54) **Medizinisches Sensorsystem zum Nachweis von zumindest einem Merkmal in zumindest einem tierischen und/oder menschlichen Körper**
Medical sensor system for detecting at least one feature in at least one animal and/or human body
Système de capteur médical pour contrôler au moins une caractéristique dans au moins un corps animal et/ou humain

(30) Priorität: 28.09.2010 US 387015 P
(43) Veröffentlichungstag der Anmeldung: 28.03.2012
(73) Patentinhaber: BIOTRONIK SE & Co. KG, 12359 Berlin (DE)
(72) Erfinder: Ulmer, Jens, 8008 Zürich (CH)
(74) Vertreter: Lindner-Vogt, Karin L.

(56) Entgegenhaltungen:
- WO-A2-2010/006592
- US-A- 6 002 954
- ANDREA PRIETO ASTALAN ET AL: "Biomolecular reactions studied using changes in Brownian rotation dynamics of magnetic particles", BIOSENSORS AND BIOELECTRONICS, ELSEVIER BV, NL, Bd. 19, Nr. 8, 15. März 2004 (2004-03-15), Seiten 945-951, XP008130202, ISSN: 0956-5663, DOI: 10.1016/J.BIOS.2003.09.005

## Beschreibung

Die Erfindung betrifft ein medizinisches Sensorsystem zum Nachweis von zumindest einem Merkmal in zumindest einem tierischen und/oder menschlichen Körper nach dem Oberbegriff des Patentanspruchs 1.

In der Medizin kommen Sensorsysteme zum Einsatz, bei denen zumindest Teile des Systems direkt in einen Körper eines Patienten eingesetzt bzw. implantiert werden, um möglichst genau und unmittelbar physiologische Ist-Zustände zu erfassen.

Bekannt ist beispielsweise aus der US 6,682, 938 B1 ein Sensorsystem, das auf einem optischen Prinzip beruhen. Hierbei ändern sich abhängig von einer Analytkonzentration die Fluoreszenzeigenschaften eines fluoreszierenden Farbstoffs, der eingebettet in einem injizierbaren Gel vorliegt. Eine Fluoreszenzänderung wird dabei durch ein Signalverarbeitungssystem außerhalb des Körpers drahtlos ausgelesen. Damit verlässliche Messergebnisse erzielt werden, muss eine aufwendige Kalibrierung vorgenommen werden. Des Weiteren werden Systeme eingesetzt, bei denen das Funktionsprinzip auf einen direkten Kontakt des Sensors mit einer Verarbeitungseinheit beruht. Hierbei müssen Komponenten des Sensors oder der Verarbeitungseinheit oft materialbedingt nach einem gewissen Zeitraum wieder explantiert werden.

Der Erfindung liegt die Aufgabe zugrunde, ein medizinisches Sensorsystem bereitzustellen, das ein einfaches und zuverlässiges Funktionsprinzip aufweist, obwohl ein Sensor und eine Verarbeitungseinheit räumlich getrennt voneinander eingesetzt werden.

Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Die Erfindung geht aus von einem medizinischen Sensorsystem zum Nachweis von zumindest einem Merkmal in zumindest einem tierischen und/oder menschlichen Körper mit zumindest einer in den tierischen und/oder menschlichen Körper implantierbaren Signalaufnahmeeinheit und mit zumindest einer räumlich von der Signalaufnahmeeinheit getrennt angeordneten Signalverarbeitungseinheit mit einem Sender und einem Empfänger.

Der Sender ist dazu ausgebildet, ein magnetisches Wechselfeld zum Einwirken auf die Signalaufnahmeeinheit auszusenden und der Empfänger ist dazu ausgebildet, ein Antwortsignal der Signalaufnahmeeinheit zu empfangen, das durch die magnetische Wechselwirkung des magnetischen Wechselfelds mit der Signalaufnahmeeinheit generierbar ist.

In diesem Zusammenhang soll unter einem "Sensorsystem" insbesondere ein System mit zumindest einer Signalaufnahmeeinheit und zumindest einer Signalverarbeitungseinheit verstanden werden, das zum Nachweis eines Merkmals eines tierischen und/oder menschlichen Körpers bzw. eines Organs oder eines Gewebes und insbesondere eines subkutanen Gewebes dient. Unter einem "Merkmal" soll insbesondere ein Parameter, wie ein pH-Wert, eine Ladung, beispielsweise eines Ions, eine Temperatur, eine Größe, eine Masse, ein Aggregatszustand, eine An-. bzw. Abwesenheit und/oder insbesondere eine Menge eines Stoffs, wie ein Salz, ein Zucker oder ein Protein, und/oder eines Analyten verstanden werden. Generell wäre jedoch auch jedes andere, dem Fachmann als zweckdienlich erscheinendes Merkmal denkbar. Bevorzugt bezieht sich das Merkmal auf einen variablen Bestandteil des tierischen und/oder menschlichen Körpers.

Unter einer "Signalaufnahmeeinheit" soll hier insbesondere eine Einheit verstanden werden, die zumindest auf einen Zustand und/oder insbesondere auf ein Signal mit einem Antwortsignal reagiert, wobei die Reaktion insbesondere eine Abhängigkeit von dem Merkmal aufweist. Die Signalaufnahmeeinheit ist zumindest zum Teil und bevorzugt komplett in den tierischen und/oder menschlichen Körper implantiert und so insbesondere in einem Zustand einer Signalaufnahme *in vivo* angeordnet. Zudem ist sie aus einem biokompatiblen Material gefertigt. Ferner stellt sie einen passiven Biosensor dar.

Des Weiteren soll unter einer "Signalverarbeitungseinheit" eine Einheit verstanden werden, die insbesondere zumindest ein Antwortsignal der Signalaufnahmeeinheit misst und/oder auswertet bzw. verarbeitet. Bevorzugt weist sie hierfür zumindest einen Empfänger auf. Zudem dient die Signalverarbeitungseinheit auch zur Generierung eines Signals in der Form eines magnetischen Wechselfelds, wofür sie zumindest einen Sender aufweist. In diesem Zusammenhang soll unter einem "magnetischen Wechselfeld" insbesondere ein magnetisches Feld verstanden werden, das in Stärke und/oder Polung wechselt und das durch Wechselspannung oder -strom hervorgerufen wird. Ferner soll unter der Wendung "zum Einwirken auf die Signalaufnahmeeinheit" insbesondere verstanden werden, dass eine Wechselwirkung zwischen dem Wechselfeld und zumindest einem Bestandteil der Signalaufnahmeeinheit stattfindet, durch die ein Antwortsignal generiert wird. Zweckmäßigerweise kann es zudem vorgesehen sein, dass die Signalverarbeitungseinheit zusätzlich mittels einer Recheneinheit eine Wertung des Merkmals, beispielsweise an einen Patienten oder einen Arzt, über eine Ausgabeeinheit ausgibt. Die Signalaufnahmeeinheit und die Signalverarbeitungseinheit sind räumlich getrennt voneinander angeordnet, wobei unter "räumlich getrennt" insbesondere verstanden werden soll, dass zwischen diesen Einheiten keine physikalische Verbindung besteht und/oder dass das Antwortsignal der Signalaufnahmeeinheit kabel- bzw. drahtlos ausgelesen werden kann. Die Signalverarbeitungseinheit ist bevorzugt als ein AC Suszeptometer ausgebildet.

Durch die erfindungsgemäße Ausgestaltung kann eine Messung mit einer vorteilhaften geringen Dämpfung im Körpergewebe erfolgen, was zu guten, exakten und zuverlässigen Messergebnissen führt. Zudem kann eine aufwendige Kalibrierung des Messsystems entfallen um so Zeit und Kosten zu sparen. Ferner kann die zu implantierende Signalaufnahmeeinheit gegenüber Vorrichtungen des Stands der Technik klein und weniger komplex gehalten werden. Auch ein Format der Signalverarbeitungseinheit kann vorteilhaft reduziert werden und handlich gestaltet werden.

Das medizinische Sensorsystem weist zumindest ein magnetisches Nanopartikel auf, das dazu ausgebildet ist, in Abhängigkeit von einer Konzentration eines Analyten entweder an ein Analogon des Analyten oder an einen Rezeptor des Analyten und des Analogon reversibel zu binden. Durch die Realisierung des funktionalisierten magnetischen Nanopartikels und der analytkonzentrationsabhänigen reversiblen Bindung, kann ein einfaches Messprinzip Bauteil und Kosten sparend eingesetzt werden. Unter "ausgebildet" soll hier speziell vorgesehen, ausgestattet, ausgelegt und/oder vorbereitet verstanden werden. Ferner soll unter einem "magnetischen Nanopartikel" in diesem Zusammenhang insbesondere ein Partikel bzw. Teilchen verstanden werden, das eine Erstreckung und/oder einen Durchmesser von zwischen 1 nm und 100 nm, bevorzugt zwischen 10 nm und 50 nm und besonders vorteilhaft von 20 nm aufweist. Zudem weist das Nanopartikel einen magnetischen Stoff, wie beispielsweise Verbindungen aus Eisen, Nickel, Kobalt, Chrom und/oder Legierungen aus Mangan mit Aluminium, Kupfer, Zinn, Antimon, Arsen, Wismut, Bor oder jeden anderen Stoff, jede andere Verbindung, jede andere Legierung, die der Fachmann für sinnvoll hält, auf. Vorteilhafterweise weist das Sensorsystem viele Nanopartikel auf und/oder vorteilhaft eine Massenkonzentration bzw. eine Masse pro Volumen Sensoreinheit zwischen 0,01-100 mg/ml bevorzugt zwischen 0,1-50 mg/ml und besonders vorteilhaft zwischen 1-10 mg/ml auf. Eine detektierbare Massenkonzentration von 1 mg/ml entspricht beispielsweise bei einem angenommenen Teilchendurchmesser von 20 nm einer Teilchenzahl von 3.3 10¹⁵. In einer weiteren vorteilhaften Ausgestaltung der Erfindung wird vorgeschlagen, dass das magnetische Nanopartikel Fe₃O₄ umfasst bzw. aus monodispersen Eisenoxid Einkristallen besteht, wodurch es besonders vielseitig einsetzbar und biokompatibel ist.

Zudem ist das Nanopartikel funktionalisiert, indem zumindest ein Teil seiner Oberfläche und bevorzugt die komplette Oberfläche beschichtet ist. Die Beschichtung umfasst entweder einen Rezeptor des nachzuweisenden Analyten bzw. eines Analogons des Analyten oder das Analogon des Analyten. Eine Anbindung der funktionalen Moleküle an die magnetischen Nanopartikel kann z.B. über nasschemische Verfahren erfolgen. In diesem Zusammenhang soll unter einem "Analyt" insbesondere das Molekül bzw. der Stoff verstanden werden, das bzw. der als Merkmal von dem Sensorsystem im tierischen und/oder menschlichen Körper nachgewiesen werden soll. Ferner soll unter einem "Analogon" ein Molekül und/oder ein Stoff verstanden werden, das/der aufgrund seiner Strukturähnlichkeit, ähnlicher Ladungsverteilung und/oder einem anderen, dem Fachmann für zweckmäßig erscheinendes Kriterium zu dem Analyten von dem gleichen Rezeptor gebunden wird. Ein "Rezeptor" definiert hier insbesondere ein Molekül und/oder einen Stoff, das/der den Analyten und durch die Ähnlichkeit des Analogons zum Analyten auch das Analogon spezifisch bindet. Dabei kann der Rezeptor aus der Stoffklasse der Peptide, Proteine - insbesondere der Antikörper und deren Fragmente, RNA, DNA - insbesondere Aptamere und deren Verwandte, zyklische Makromoleküle - wie insbesondere Moleküle, die selektiv Ionen binden bzw. Ionophore, nichtzyklische Makromoleküle - d.h. Polymere deren Erkennungsmerkmal während einer Polymerisation eingeprägt wurde bzw. sogenannte "molecular imprinted polymers" - und/oder jeder anderen, dem Fachmann für sinnvoll erscheinenden Stoffklasse sein. Unter "reversibel" soll hier insbesondere umkehrbar bzw. (auf)lösbar verstanden werden.

Zudem ist vorgesehen, dass die Signalaufnahmeeinheit im Fall des mit Analogon beschichteten Partikels zusätzlich den Rezeptor aufweist und im Falle des mit Rezeptor beschichteten Teilchens das Analogon. Hierbei sind diese an einer festen Struktur, wie beispielsweise einer Netz- oder Gitterstruktur, angeordnet. Durch die Anwesenheit dieser Gegenspieler kommt es zu einer reversiblen Bindung zwischen dem Analogon und dem Rezeptor. Der Nachweis des Analyten beruht nun auf einer Verdrängungsreaktion, bei dem der Analyt das Analogon vom Rezeptor verdrängt bzw. auf einer kompetitiven Bindung des Analyten an den Rezeptor. Ändert sich im untersuchten Gewebe die lokale Konzentration des Analyten, wird die Anzahl der Bindungen zwischen dem Analogon und dem Rezeptor reduziert und eine Bewegungs- oder Rotationsfreiheit und/oder insbesondere eine magnetische Relaxation des funktionalisierten magnetischen Nanopartikels, das zuvor durch die Bindung des Analogons an dem Rezeptor relativ gegenüber der festen Struktur fixiert war, gegenüber der festen Struktur verändert bzw. erhöht. Diese kann mit einem magnetischen Wechselfeld mit einer magnetischen Flussdichte mit beispielsweise einer Amplitude von 0,5 mT gemessen werden.

Der Analyt ist bevorzugt ein Kohlenhydrat und/oder ein Polysaccharid. Grundsätzlich wäre jedoch auch jede andere, dem Fachmann für anwendbar erscheinende Substanzklasse, wie Peptide, Hormone, Proteine, Fette, DNA, RNA, Nucleotide, Ionen, pH etc. denkbar. Erfindungsgemäß ist die Signalaufnahmeeinheit jedoch als Sensor für den Analyten Glucose ausgebildet, wodurch eine Ist-Konzentration an Glucose besonders konstruktiv einfach und schnell bestimmt werden kann.

Zweckmäßigerweise ist/sind an der Oberfläche des Nanopartikels als Rezeptor ein und/oder mehrere Lectine chemisch gebundenen. Alternativ können auch Kohlenhydrate bzw. Polysaccharide gebunden sein. Folglich sind als Analogon Kohlenhydrate bzw. Polysaccharide und Lectine an der festen Struktur angeordnet.

Ferner ist es vorteilhaft, wenn das Analogon von einem Dextran und/oder der Rezeptor von Concanavalin-A gebildet ist, wodurch das Nanopartikel mit Concanavalin-A beschichtet ist. Alternativ wäre auch die umgekehrte Anordnung möglich. Durch die Dextran Concanavalin-A-Paarung kann das Sensorsystem besonders selektiv auf den Analyten Glucose abgestimmt werden.

Ein besonders robuster Glucosenachweis kann bereitgestellt werden, wenn eine Konzentration von Glucose ein Bindungsverhalten eines magnetischen und mit Concanavalin-A beschichteten Nanopartikels an Dextran beeinflusst und damit ein Relaxationsverhalten des Nanopartikels ändert.

Ferner weist die Signalaufnahmeeinheit zumindest ein Hydrogel auf, das von vernetzten Dextranmolekülen gebildet ist, wobei in diesem Zusammenhang unter einem "Hydrogel" insbesondere ein Gel aus einem Polymer bzw. Copolymer verstanden werden soll, das Wasser enthält, aber wasserunlöslich ist. Ferner sind dessen Moleküle chemisch, z. B. durch kovalente oder ionische Bindungen, oder physikalisch, z. B. durch Verschlaufen von Polymerketten, zu einem dreidimensionalen Netzwerk verknüpft. Die Dextranmoleküle weisen ein Gewicht zwischen 1 kDa und 500 kDa, bevorzugt zwischen 10 kDa und 200 kDa und besonders vorteilhaft zwischen 15 kDA und 25 kDa auf. Das Hydrogel hat bevorzugt eine Konsistenz, sodass es einfach subkutan injiziert werden kann. Hierbei wird das Hydrogel bevorzugt als Dispersion von 200 bis 300 µm großen Gelkügelchen suspendiert in einer zu Injektionszwecken geeigneten Lösung unter der Haut platziert. Grundsätzlich wäre es jedoch auch möglich, dass das Hydrogel erst *in situ* beispielsweise durch einen Temperaturwechsel geliert. Durch das Hydrogel kann die Signalaufnahmeeinheit bzw. der Biosensor besonders einfach und anwenderfreundlich mittels Injektion ins Gewebe platziert werden. Zudem treten patientenfreundlich und gesundheitsfördernd sehr geringe bis keine Fremdkörperreaktion nach der Implantation auf. Des Weiteren bleiben durch die reduzierte bis ausbleibende Fremdkörperreaktion bzw. Entzündungsreaktion Bedingungen an der Implantationsstelle unbeeinflusst, wodurch optimale Nachweisbedingungen herrschen.

Besonders vorteilhaft ist das Hydrogel von einem Copolymer gebildet, wobei hier unter einem "Copolymer" ein Polymer verstanden werden soll, das verschiedene Polymertypen enthält, also beispielsweise zusätzlich zu Dextranmolekülen zumindest einen anderen Molekülbaustein und einen dem Fachmann bekannten Vernetzungsgrad aufweist. Dadurch können konstruktiv einfach neben der Rezeptoreigenschaft weitere Eigenschaften, wie eine erhöhte Biokompatibilität, eine bessere Abbaubarkeit, eine vereinfachte Injizierbarkeit und/oder eine optimierte *ex vivo* Stabilität in das Hydrogel bzw. die Signalaufnahmeeinheit integriert werden.

Ferner ist in dem Hydrogel zumindest ein magnetisches Nanopartikel bzw. sind in dem Hydrogel Nanopartikel angeordnet. Die Nanopartikel sind bevorzugt homogen über das Hydrogel verteilt. Generell wäre auch eine inhomogene Verteilung denkbar. Die Nanopartikel können insbesondere reversibel an das Hydrogel bzw. Strukturen des Hydrogels binden. Durch die Anordnung der bevorzugt mit Concanavalin-A beschichteten Nanopartikel in dem Hydrogel und bevorzugt in einem Dextran-Copolymer-Hydrogel kann die Fixierung der Nanopartikel durch die Rezeptor-Analogon-Interaktion besonders einfach realisiert werden.

Bei der alternativen Ausgestaltung der Nanopartikel mit einer Dextranbeschichtung als Analogon kann auf Dextran im Copolymergerüst teilweise oder ganz verzichtet werden. Hingegen muss an das Hydrogel in einer geeigneten Weise das Concanavalin-A als Rezeptor chemisch gebunden werden.

Die Signalverarbeitungseinheit ist vorteilhaft mit einem Abstand in unmittelbarer Nähe zu der Signalaufnahmeeinheit angeordnet. Sie sollte aber ausreichend weit entfernt sein, um nicht implantiert zu werden, wodurch sie vorteilhaft ex *vivo* bzw. extern vom Körper angeordnet ist. Dadurch kann bei der Signalaufnahmeeinheit Platz und Gewicht sparend auf eine interne Energieversorgung verzichtet werden. Ferner wird so die für die Messung notwendige Energie über das extern angelegte magnetische Wechselfeld drahtlos zugeführt.

Besonders komfortabel kann die Signalverarbeitungseinheit gestaltet werden, wenn sie sich in einem externen Gerät, wie einem Laptop oder einem Mobiltelefon, oder einem Gegenstand bzw. Gebrauchsgegenstand, wie einem Schmuckstück, einem Schlüsselanhänger, einer Brille oder bevorzugt in einer Armbanduhr befindet. Durch eine Integration in einer Armbanduhr kann sie besonders kompakt und klein ausgeführt werden.

Zweckmäßigerweise ist die Signalaufnahmeeinheit zumindest teilweise und zwar von zumindest zu 50%, bevorzugt zu 75% und besonders bevorzugt zu 100% von einer biodegradierbaren Schicht umgeben. Hierbei ist in einem Kern der Schicht bevorzugt die Signalaufnahmeeinheit mit dem Dextran-Hydrogel und den mit Rezeptor beschichteten magnetischen Nanopartikeln angeordnet. Die biodegradierbare Schicht ist bevorzugt aus Polymeren, die aus Vinylester, Acrylaten, Methacrylaten, Milchsäure, Glycolsäure und/oder anderen Lactonen oder N-Isopropylamid bestehen, aufgebaut. Hierbei ist zu beachten, dass diese Polymerschicht zumindest weitestgehend, also zu mehr als 90%, durchlässig für den Analyten ist. Durch diesen Schale-Kern Aufbau der Signalaufnahmeeinheit kann diese sowohl ideal auf ihre Funktion der Analyt-Rezeptor-Analogon-Bindung ausgelegt werden, als auch auf eine Biokompatibilität und/oder Biodegradierbarkeit.

Vorteilhaft weist die Signalaufnahmeeinheit eine vollständige Biodegradierbarkeit auf, wodurch die Signalaufnahmeeinheit nach einem Funktionsverlust gesundheitsfördernd und patientenfreundlich nicht wieder explantiert werden muss. Diese Degradation und insbesondere eine zeitlich kontrollierte proteolytische Biodegradation des Hydrogels kann vorteilhaft weiter verbessert werden, indem ein Copolymer verwendet wird, das biodegradierbare Polymerketten aufweist, an die Dextranmoleküle mit niedrigem Molekulargewicht (20 kDa) angebunden sind. Diese Polymerketten können aus Acrylaten, Methacrylaten, Vinylestern, Carbonaten, Caprolactonen, Milchsäure, Glycolsäure und/oder aus jedem weiteren, dem Fachmann für sinnvoll erscheinenden Molekül zusammengesetzt sein. Ferner kann es vorgesehen sein, dass veränderlichen Spacer, beispielsweise aus Polyethylenglycol, Polypropylenglycol o.ä., in die Polymerkette eingebaut sind. Eine Polymerisation kann beispielsweise mit thermischen oder lichtinduzierten Radikalstartern initiiert werden. Durch diese Zusammensetzung kann eine Abbaugeschwindigkeit im lebenden Organismus konstruktiv einfach über hydrophile und hydrophobe Eigenschaften der Spacer eingestellt werden.

Ferner ist es zweckmäßig, wenn die Signalaufnahmeeinheit mittels eines externen Stimulus abbaubar gemacht werden kann bzw. wenn die Biodegradierbarkeit von außen ausgelöst werden kann. Hierbei können chemische Stimuli, wie beispielsweise eine Änderung des pH Werts, verursacht durch Zuführen einer schwachen Säure oder Hervorrufen einer Entzündungsreaktion, in Betracht gezogen werden. So reagieren Polymere beispielsweise aufgebaut aus Acrylsäure, Methacrylsäure, Maleinanhydrid, N,N-dimethylaminoethylmethacrylat sensitiv auf eine pH-Änderung. Diese Veränderung bewirkt eine Änderung der Löslichkeit des Polymergerüsts und dementsprechend die Abbaubarkeit des Hydrogels.

Bevorzugt hat jedoch der externe Stimulus einen physikalischen Ursprung, wie beispielsweise eine Änderung der Temperatur, wodurch der Stimulus mit einem konstruktiv einfachen Prinzip generiert werden kann. Hierbei kann ein Copolymersystem als Hydrogel verwendet werden, bei dem thermoresponsive Polymereinheiten an die niedermolekulare Dextraneinheiten angebunden sind. Dabei handelt es sich bei den thermoresponsiven Einheiten vorzugsweise um Polymere, die aus folgenden Monomeren synthetisiert werden können: N-isopropylacrylamid, N,N-diethylacrylamid, Methylvinylether, N-vinylcaprolactam, Ethyleneoxid-co-propylenoxid. Diese ändern in Abhängigkeit der Temperatur die Lösungseigenschaften des Copolymers in einem wässrigen Medium und können demzufolge das Hydrogel gezielt einer Biodegradation zugänglich machen. Weiterhin können Polymereinheiten eingebaut werden, die das Quellverhalten und die Biokompatibilität steuern. Hier können Poly-Milch-Glycolsäure, Polyethylenglyol, Polypropylenglycol und Polymere hergestellt aus Acrylaten, Methacrylaten oder Vinylester beispielhaft genannt werden. Die Aufzählung an Substanzen ist hier nicht abschließend. Der Fachmann wird die Liste nach seinem Fachwissen um Substanzen mit ähnlichen Eigenschaften ergänzen.

Somit ist eine Ausgestaltung der Signalaufnahmeeinheit besonders vorteilhaft, wenn diese zumindest ein Polymer aus Monomeren der Gruppe: N-Isopropylacrylamid, N,N-Diethylacrylamid, Methylvinylether, N-Vinylcaprolactam, Ethylenoxid-co-Propylenoxid, Milchsäure, Glycolsäure, Trimethylcarbonat, Acrylat, Methacrylat, Hydoxyethylen-Metacrylat, Vinylester aufweist, wodurch eine Stimulation der zeitlichen und thermischen Biodegradierbarkeit leicht erreicht werden kann. Hierbei kann jede, dem Fachmann für zweckdienlich erscheinende Kombination der Monomere mit und ohne Dextran in Frage kommen.

Des Weiteren kann es vorteilhaft sein, wenn eine Biodegradierbarkeit thermisch mittels Anlegen eines starken magnetischen Wechselfelds stimulierbar ist. Unter einem "starken magnetischen Wechselfeld" soll hier insbesondere ein Feld verstanden werden, das über einen Zeitraum von weniger als 30 Minuten, bevorzugt weniger als 20 Minuten, besonders bevorzugt von weniger als 15 Minuten und insbesondere von mindestens 10 Minuten auf die Nanopartikel mit einer magnetischen Feldstärke H von weniger als 100 kA/m, bevorzugt weniger als 50 kA/m, besonders bevorzugt von weniger als 20 kA/m und insbesondere bei 10 kA/m bei einer Frequenz von 300-400 kHz einwirkt. Dabei wandeln die Nanopartikel das eingestrahlte starke magnetische Wechselfeld in Wärme um, wobei durch die Temperaturänderung die oben beschriebene Veränderung der Löslichkeit eintritt.

Bei einer Feldstärke H von ca. 10 kA/M bei ca. 350 kHz kann es beispielsweise zu einer Heizleistung von 100 W/g Eisenpartikel kommen. Da es sich bei den thermoresponsiven Polymeren um reversible Konformationsänderungen handelt, scheint eine Einwirkungszeit von 10 Minuten sinnvoll, sodass es durch das Kollabieren des Polymergerüsts und dem Aufheizen des umliegenden Gewebes zu einer milden Entzündungsreaktion kommt, was die Hydrogeldegradation beschleunigt. Zudem kann ferner durch die Temperaturerhöhung der Rezeptor, wie bspw. das ConA, inaktiviert und die Bindungsfähigkeit zum Analyten bzw. zum Analogon bzw. zu Dextran/Glucose vermindert werden. Somit kann es auch hier zu einer Phasenseparation der Hydrogelkomponenten kommen, was die Degradation wiederum beschleunigt. Durch die erfindungsgemäße Ausgestaltung kann Platz, Kosten, Bauteil und Bauraum sparend auf eine separate Vorrichtung zur Stimulation der Degenerierbarkeit verzichtet werden, da hierfür die Signalverarbeitungseinheit eingesetzt werden kann.

In einer weiteren Ausgestaltung der Erfindung ist vorgesehen, dass ein funktionalisiertes magnetisches Nanopartikel dazu vorgesehen ist, an ein im Körper implantiertes Hydrogel reversibel zu binden und diese Bindung ein Relaxationsverhalten des Nanopartikels im magnetischen Wechselfeld bestimmt. Unter "funktionalisiert" soll hier insbesondere modifiziert und/oder besonders vorteilhaft beschichtet verstanden werden. Dadurch kann eine Menge an Analyt besonders einfach und zuverlässig ermittelt werden.

Zudem geht die Erfindung aus von einem Verfahren zum Auslesen eines medizinischen Sensorsystems, wobei vorgeschlagen wird, dass von einem Sender einer Signalverarbeitungseinheit ein magnetisches Wechselfelds zum Einwirken auf eine Signalaufnahmeeinheit ausgesendet wird und von einem Empfänger der Signalverarbeitungseinheit ein Antwortsignal der Signalaufnahmeeinheit empfangen wird, das durch die magnetische Wechselwirkung des magnetischen Wechselfelds mit der Signalaufnahmeeinheit generiert wird.

Eine einfache Detektion und Messung kann erfolgen, wenn das Antwortsignal von einem Relaxationssignal zumindest eines Nanopartikels gebildet wird. Hierbei kann eine Messung der Relaxationszeit vorteilhaft inhärent erfolgen und ist unabhängig von einer eingestrahlten Energie. Ferner kann hier mit einem einfachen Referenzsystem gearbeitet werden. Bevorzugt wird das Relaxationssignal durch eine Änderung eines Bindungsverhaltens zwischen dem zumindest einen Nanopartikel und einem Bestandteil eines Hydrogels verursacht, wobei das Bindungsverhalten abhängig von einer Konzentration eines Analyten ist. Die Veränderung des Bindungsverhaltens kann durch Anlegen eines externen magnetischen Wechselfeldes bestimmt werden. Liegt das Nanopartikel ungebunden bzw. frei vor, ist die komplexe Suszeptibilität höher mit beispielsweise einem Max. von 50 kHz als die eines gebundenen bzw. nicht mehr frei beweglichen Teilchens, wo das Max. beispielsweise bei 20 Hz liegt. Die Zahlenwerte stellen hier lediglich Beispiele dar. Der Fachmann wird sie selbsttätig nach den technischen Gegebenheiten des verwendeten Systems ermitteln. Dieser Unterschied liegt darin begründet, dass bei freien Partikeln eine isotrope Reorientierung der Partikel nach einem Abschalten des Magnetfelds über die Brownsche-Relaxation stattfindet, wohingegen es bei gebundenen Partikeln zu einer Neel-Relaxation kommt. Da dies zwei unterschiedliche Mechanismen darstellen, sind auch die Zeitkonstanten, während denen es zu der isotropen Reorientierung kommt, unterschiedlich (µs gegenüber s). Dies schlägt sich signifikant in der Resonanzfrequenz der Messung nieder. Durch das analytkonzentrationsabhängige Bindungsverhalten kann ein Mechanismus bereitgestellt werden, der das Relaxationssignal direkt proportional beeinflusst, wodurch dieses konstruktiv besonders einfach realisierbar ist.

Vorteilhaft wird nach einer vorbestimmten Zeit die Signalaufnahmeeinheit durch ein thermisch gesteuertes Signal biodegradierbar, wobei hier die Zeit konstruktiv einfach mittels in das Hydrogel eingebundener Moleküle gesteuert werden kann. Hierbei dient das Signal als Trigger zur Auslösung des Degradationsprozesses, der im Anschluss daran stattfindet und mehrere Wochen andauern kann. Bevorzugt ist die Signalaufnahmeeinheit bzw. das Hydrogel in einem Zeitraum von weniger als einem Jahr, bevorzugt von weniger als einem halben Jahr, besonders bevorzugt von weniger als 12 Wochen und insbesondere in 6 bis 8 Wochen vollständig abgebaut. Zudem soll mit diesem Signal die Funktionalität der Signalaufnahmeeinheit auf kleiner 5% herabgesetzt werden.

Weiterhin sind noch Systeme zu erwähnen, die nicht Bestandteil der Erfindung sind, die es z.B. erlauben Ionenkonzentrationen im subkutanen Gewebe selektiv nachzuweisen. Dazu werden sowohl am Hydrogel als auch am magnetischen Nanopartikel als Rezeptor bzw. Analogon Kryptanten angebracht. Diese binden erst durch Zusammenlagerung von zwei und/oder mehr Kryptantmoleküleinheiten das entsprechende Ion, den Analyten. Hierzu kann exemplarisch ein kaliumselektiver Sensor beschrieben werden, bei dem Kronenether sowohl in der Polymermatrix als auch auf dem mag. Nanopartikel vorhanden sind. Durch eine 2:1 Komplexierung (2 Kronenetherringe komplexieren 1 Ion) wird dann eine Bindung zwischen Nanopartikel und Polymergerüst hergestellt, was sich wiederum auf das Relaxationsverhalten der Nanopartikel auswirkt.

Die Erfindung ist nachfolgend beispielhaft, anhand in Zeichnungen dargestellter Ausführungsbeispiele, näher erläutert. Es zeigen:
- Fig. 1A: einen Mensch mit einem erfindungsgemäßen teilweise implantierten Sensorsystem in einer schematischen Darstellung,
- Fig. 1B: das Sensorsystem der Fig. 1a in einer schematischen Detaildarstellung,
- Fig. 2A: ein Nanopartikel eingebettet in einem Hydrogel bei einer geringen Analytkonzentration in einer schematischen Darstellung,
- Fig. 2B: das Nanopartikel der Fig. 2a bei einer hohen Analytkonzentration in einer schematischen Darstellung,
- Fig. 3: ein Abbaudiagramm der Signalaufnahmeeinheit der Fig. 1b,
- Fig. 4: eine alternative Signalaufnahmeeinheit mit einer die Signalaufnahmeeinheit ummantelnden Schicht und
- Fig. 5: eine weitere alternative Signalaufnahmeeinheit einem dextranbeschichteten Nanopartikel.
In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verweisen.

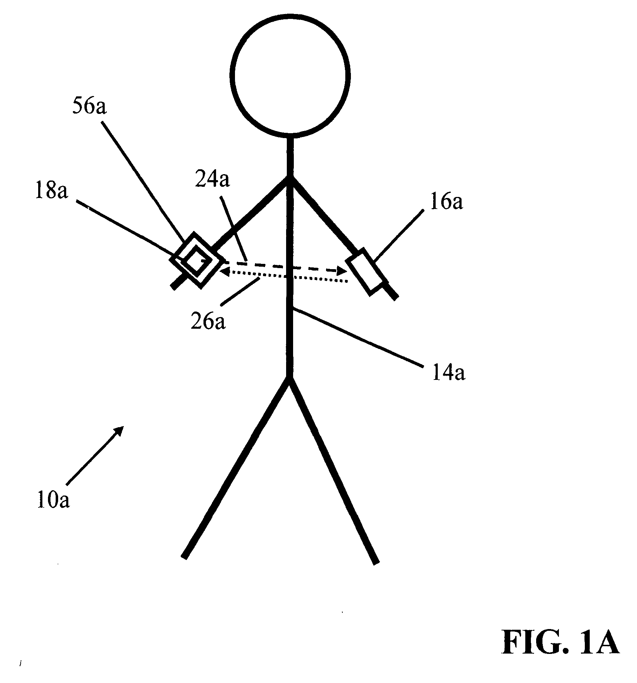

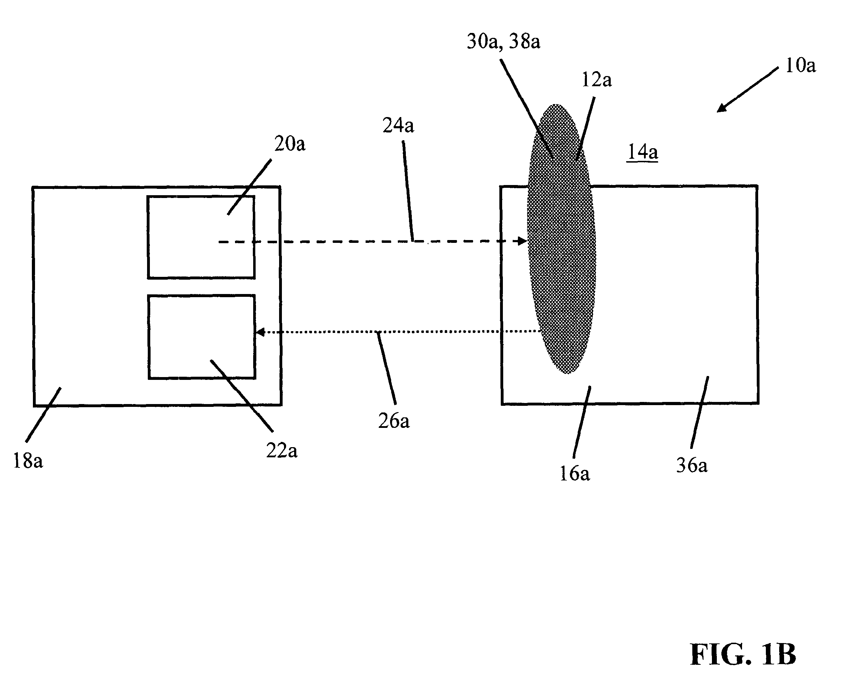

Die Fig. 1A zeigt ein medizinisches Sensorsystem 10a zum Nachweis von einem Merkmal 12a in einem menschlichen Körper 14a. Das Merkmal 12a stellt eine Menge an einem Analyten 30a in der Form von Glucose 38a dar. Somit ist das Sensorsystem 10a als Sensor 36a für Glucose 38a ausgebildet (siehe Fig. 1B). Das Sensorsystem 10a weist eine in den menschlichen Körper 14a im Subkutangewebe direkt unter der Haut am Unterarm implantierte Signalaufnahmeeinheit 16a und eine Signalverarbeitungseinheit 18a in der Form eines AC Suszeptometers auf. Die Signalverarbeitungseinheit 18a ist ex *vivo* in einer Armbanduhr 56a, räumlich getrennt von der Signalaufnahmeeinheit 16a, angeordnet. Ferner weist die Signalverarbeitungseinheit 18a einen Sender 20a und einen Empfänger 22a auf, die in der Fig. 1B zur besseren Darstellung vergrößert abgebildet sind.

Der Sender 20a ist dazu ausgebildet, ein magnetisches Wechselfeld 24a mit einer magnetischen Flussdichte und einer Amplitude von z.B. 0.5 mT zum Einwirken auf die Signalaufnahmeeinheit 16a auszusenden. Der Empfänger 22a wiederum ist dazu ausgebildet, ein Antwortsignal 26a der Signalaufnahmeeinheit 16a zu empfangen. Dieses Antwortsignal 26a wird durch die magnetische Wechselwirkung des magnetischen Wechselfelds 24a mit der Signalaufnahmeeinheit 16a generiert.

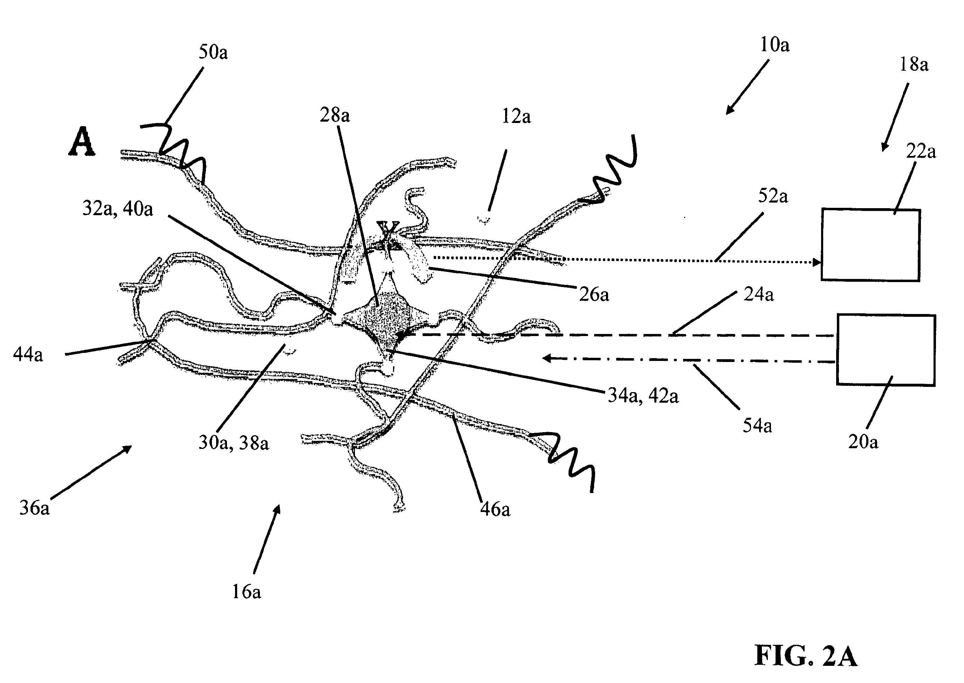

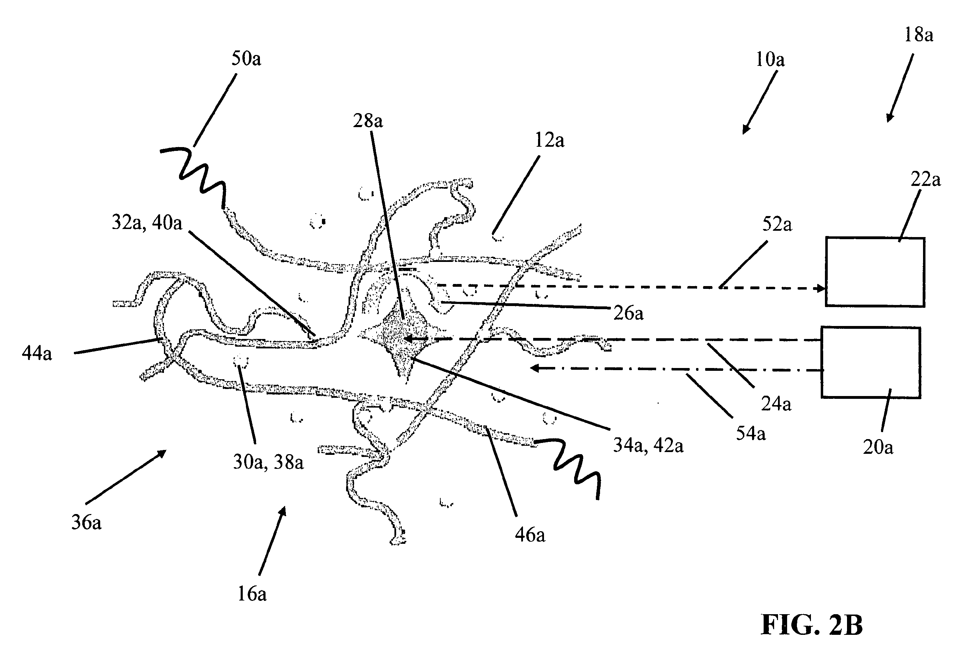

Wie in Fig. 2A gezeigt ist, weist die Signalaufnahmeeinheit 16a hierfür magnetische Nanopartikel 28a aus Fe₃O₄ mit einem Durchmesser von 20 nm auf, die in einem Hydrogel 44a angeordnet bzw. eingebettet sind. Das Hydrogel 44a ist aus einem Dextran-Copolymer 46a gebildet, das zusätzlich zu Molekülketten aus Dextran 40a mehrere Polymere 50a aus Poly-N-Isopropylacrylamid aufweist, hierdurch kann die thermoresponsible Komponente auch direkt als Crosslinker verwendet werden. Dargestellt sind Dextranmolekülketten, wobei zum besseren Verständnis vereinzelt die bindungsrelevanten bzw. in der Fig. 2b die kettenendseitigen Dextranmoleküle als Punkte dargestellt sind.

Die Nanopartikel 28a sind funktionalisiert indem sie mit einem Rezeptor 34a des Analyten 30a beschichtet sind. Der Rezeptor 34a ist von dem Lectin Concanavalin-A 42a gebildet. Durch die Ähnlichkeit bzw. Übereinstimmung von Glucose 38a mit Dextran 40a bindet der Rezeptor 34a Concanavalin-A 42a auch an Dextran 40a, wodurch dieses ein Analogon 32a zu dem Analyten 30a Glucose 38a darstellt. Die beschichteten Nanopartikel 28a sind also befähigt sowohl an Dextran 40a und somit an das Hydrogel 44a als auch an Glucose 38a zu binden. Diese Bindungsfähigkeit ist reversibel, wodurch der Bindungszustand des Rezeptors 34a von der An- bzw. Abwesenheit und damit der Konzentration des Analyten 30a abhängig ist.

Ändert bzw. erhöht sich nun am Implantationsort der Signalaufnahmeeinheit 16a die lokale Konzentration von Glucose 38a, wie dies in Fig. 2B gezeigt ist, wird die Anzahl der Rezeptor-Analogon- 34a-32a bzw. Concanavalin-A-Dextran- 42a-40a Bindungen der funktionalisierten magnetischen Nanopartikel 28a zum Polymergerüst des Hydrogels 44a durch eine kompetitive Bindung von Glucose 38a an Concanavalin-A 42a reduziert. Dies führt dazu, dass eine Rotationsfreiheit der nun freien Nanopartikel 28a erhöht wird. Diese kann mit dem magnetischen Wechselfeld 24a bzw. mit dem AC Suszeptometer gemessen werden, da gebundenen Nanoartikeln 28a gegenüber freien Nanoartikeln 28a keiner Brownschen Relaxation sondern einer Neel-Relaxation unterliegen. Da dies zwei unterschiedliche Mechanismen darstellt, sind auch die Zeitkonstanten, während denen es zu einer isotropen Reorientierung kommt, unterschiedlich (µs gegenüber s). Dies führt zu signifikant unterschiedlichen Resonanzfrequenzen, die gemessen werden können.

Somit beeinflusst die Konzentration von Glucose 38a das Bindungsverhalten der magnetischen und mit Concanavalin-A 42a beschichteten Nanopartikel 28a an Dextran 40a und ändert damit ein Relaxationsverhalten der Nanopartikel 28a. Das magnetische Wechselfeld 24a erzeugt also in der Signalaufnahmeeinheit 16a ein Antwortsignal 26a in der Form eines Relaxationssignals 52a, das von dem Empfänger 22a der Signalverarbeitungseinheit 18a zu einer Aussage über die Menge an Glucose 38a am Implantationsort zum Zeitpunkt der Messung verarbeitet wird. Diese Information könnte auch über eine Ausgabeeinheit ausgegeben werden.

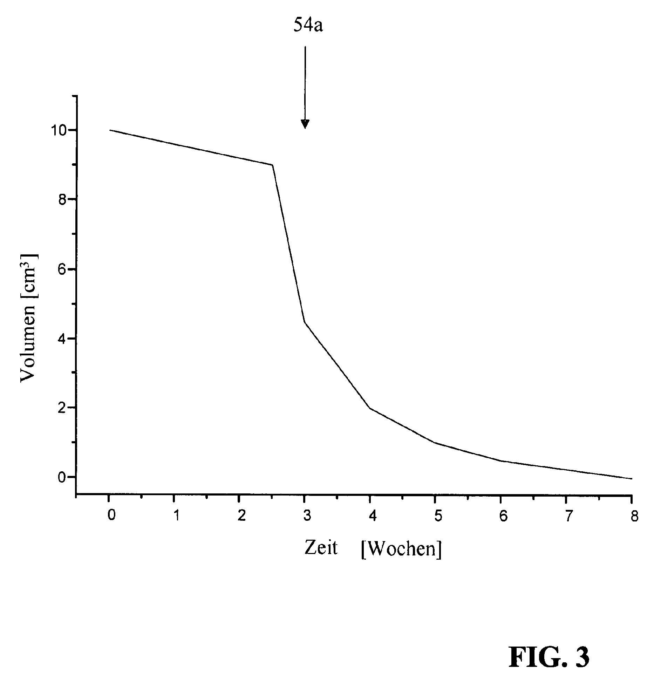

Ferner weist die implantierte Signalaufnahmeeinheit 16a durch die gezielte Wahl der Zusammensetzung des Copolymers 46a des Hydrogels 44a bzw. des Polymers 50a eine vollständige Biodegradierbarkeit auf. Diese kann, wie in Fig. 3 gezeigt, nach einer gewissen Einsatzdauer, wie beispielsweise drei Wochen nach der Implantation des Sensors 36a, durch ein externes Signal 54a stimuliert werden. Dieses Signal 54a ist ein thermisches, das durch Anlegen eines starken magnetischen Wechselfelds 24a von beispielsweise 10 kA/m bei einer Frequenz von 350 kHz ausgelöst werden kann. Dies führt dazu, dass die magnetischen Nanopartikel 28a das eingestrahlte Magnetfeld in Wärme umwandeln. Durch die Temperaturerhöhung vollzieht sich eine Phasenseparation und das Hydrogel 44a schrumpft und wird anschließend durch eine lokal generierte Entzündungsreaktion (deren Ursache in der Temperaturerhöhung zu sehen ist) über einen Zeitraum von weiteren fünf Wochen vollständig abgebaut. Wobei zu beachten ist, dass der Funktionsverlust der Signalaufnahmeeinheit 16a sofort einzutreten hat. Dieser beschriebene Aufbau erlaubt es, die Signalaufnahmeeinheit 16a nach deren Einsatzzeit und Funktionsverlust gezielt abzubauen, ohne dabei diese Einheit explantieren zu müssen.

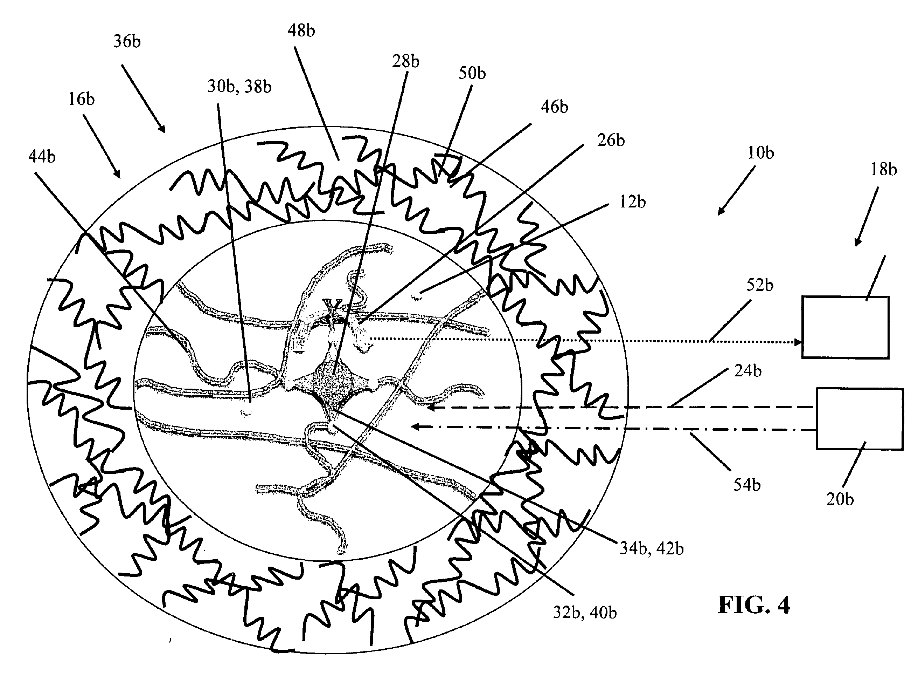

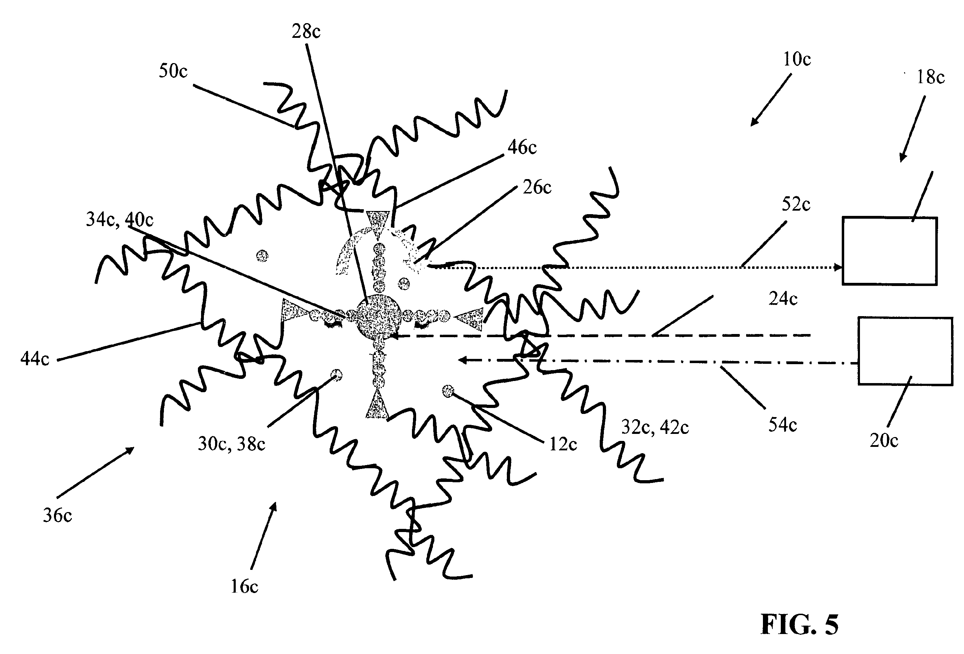

In den Fig. 4 und 5 sind alternative Ausführungsbeispiele der Signalaufnahmeeinheit 16a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele sind jedoch den Bezugszeichen der Ausführungsbeispiele die Buchstaben a bis c hinzugefügt. Die nachfolgende Beschreibung beschränkt sich im Wesentlichen auf die Unterschiede zu dem Ausführungsbeispiel in den FIG 1 bis 3, wobei bezüglich gleich bleibender Bauteile, Merkmale und Funktionen auf die Beschreibung des Ausführungsbeispiels in den FIG 1 bis 3 verwiesen werden kann.

In der Fig. 4 ist eine alternative implantierbare Signalaufnahmeeinheit 16b eines medizinischen Sensorsystem 10b zum Nachweis von einem Merkmal 12b in einem hier nicht näher dargestellten menschlichen Körper gezeigt. Die Signalaufnahmeeinheit 16b wirkt in gleicher Weise wie in den Figuren 1 bis 3 beschrieben mit einer von ihr räumlich getrennten und *ex vivo* angeordneten Signalverarbeitungseinheit 18b mit einem Sender 20b und einem Empfänger 22b zusammen.

Die Signalaufnahmeeinheit 16b unterscheidet sich von der Signalaufnahmeeinheit 16a der ersten Ausführung indem sie eine biodegradierbare Schicht 48b aufweist, die die Signalaufnahmeeinheit 16b komplett umgibt bzw. umhüllt. Die Schicht 48b besteht aus einem Gerüst aus Copolymer 46b mit Polymeren 50b zusammengesetzt aus Poly-L-Glycol-L-Milchsäure (PGLA) und Poly-N-isopropylenacrylamid (PNIPAAm). Die genaue Zusammensetzung wird der Fachmann aufgrund seines Fachwissens selbständig bestimmen. Durch diesen Aufbau ist die Schicht 48b durchlässig für einen Analyten 30b in der Form von Glucose 38b. Ferner bildet die Schicht 48b eine Schale um einen Kern, der ein Hydrogel 44b aufweist, das von Ketten aus dem Analogon 32b Dextran 40b gebildet ist und in dem Nanopartikeln 28b eingebettet sind, die mit dem Rezeptor 34b Concanavalin-A 42b beschichtet sind. Auch die Schicht 48b ist nach einer vorbestimmten Zeit durch ein thermisch gesteuertes Signal 54b biodegradierbar.

Die Fig. 5 zeigt eine weitere alternative implantierbare Signalaufnahmeeinheit 16c eines medizinischen Sensorsystem 10c zum Nachweis von einem Merkmal 12c in einem hier nicht näher dargestellten menschlichen Körper. Die Signalaufnahmeeinheit 16c wirkt in gleicher Weise wie in den Figuren 1 bis 3 beschrieben mit einer von ihr räumlich getrennten und *ex vivo* angeordneten Signalverarbeitungseinheit 18c mit einem Sender 20c und einem Empfänger 22c zusammen.

Die Signalaufnahmeeinheit 16c unterscheidet sich von der Signalaufnahmeeinheit 16c der ersten Ausführung durch die umgekehrte Anordnung eines Rezeptors 34c und eines Analogons 32c eines Analyten 30c. Die Signalaufnahmeeinheit 16c weist ein Hydrogel 44c auf, das sich aus einem Gerüst aus Copolymeren 46c mit Polymeren 50c, bestehend aus Poly-Hydroxyethyl-Metacrylat, Poly-L-Glycol-L-Milchsäure und Poly-N-isopropylenacrylamid Bestandteilen (PolyHEMA-Co-PGLA und PNIPAAm), zusammensetzt und in dem Nanopartikel 28c eingebettet sind. Die jeweiligen Gewichtsanteile der Bestandteile der Polymere 50c wird der Fachmann nach seinen Fachkenntnissen selbständig auswählen. Diese Nanopartikel 28c sind funktionalisiert indem sie als Analogon 32c zu dem Analyten 30c Glucose 38c eine Beschichtung aus Dextran 40c aufweisen. Bei dieser Ausführungsform wurde auf Dextran in dem Copolymergerüst ganz verzichtet. Hingegen ist als Gegenspieler zu der Glucose 38c und dem am Nanopartikel 28c gebundenen Dextran 40c ein Rezeptor 34c der beiden Moleküle 38c, 40c in der Form von Concanavalin-A 42c chemisch an das Hydrogel 44c gebunden.

## Patentansprüche

1. Medizinisches Sensorsystem (10a - 10c) zum Nachweis von zumindest einem Merkmal (12a - 12c) in zumindest einem tierischen und/oder menschlichen Körper (14a - 14c) mit zumindest einer in den tierischen und/oder menschlichen Körper (14a - 14c) implantierbaren, aus einem biokompatiblen Material bestehenden, passiven Signalaufnahmeeinheit (16a - 16c) die als Sensor (36a - 36c) für die Konzentration des Analyten (30a - 30c) Glucose (38a - 38c) ausgebildet ist und zumindest ein Hydrogel (44a - 44c) aufweist, das von einem Copolymer (46a - 46c) gebildet ist und in dem zumindest ein magnetisches Nanopartikel (28a - 28c) angeordnet ist und die einen passiven Biosensor bildet, und mit zumindest einer räumlich von der Signalaufnahmeeinheit (16a - 16c) getrennt angeordneten Signalverarbeitungseinheit (18a - 18c) mit einem Sender (20a - 20c) und einem Empfänger (22a - 22c), **dadurch gekennzeichnet, dass** der Sender (20a - 20c) dazu ausgebildet ist, ein magnetisches Wechselfeld (24a - 24c) zum Einwirken auf die Signalaufnahmeeinheit (16a - 16c) auszusenden und das magnetische Nanopartikel (28a - 28c), dazu ausgebildet ist, in Abhängigkeit von einer Konzentration eines Analyten (30a - 30c) entweder an ein Analogon (32a - 32c) des Analyten (30a - 30c) oder an einen Rezeptor (34a - 34c) des Analyten (30a - 30c) und des Analogons (32a - 32c) reversibel zu binden und diese Bindung ein Relaxationsverhalten des magnetischen Nanopartikels (28a - 28c) im magnetischen Wechselfeld (24a - 24c) bestimmt wobei das Analogon (32a - 32c) von einem Dextran (40a - 40c) und der Rezeptor (34a - 34c) von Concanavalin-A (42a - 42c) gebildet und Analogon (32a - 32c) oder Rezeptor (34a - 34c) Bestandteil des Hydrogels sind oder eine Beschichtung des Nanopartikels bilden und der Empfänger (22a - 22c) dazu ausgebildet ist, ein Antwortsignal (26a - 26c) der Signalaufnahmeeinheit (16a - 16c) zu empfangen, das von einem Relaxationssignal (52a - 52c) zumindest eines magnetischen Nanopartikels (28a - 28c) gebildet wird.

2. Medizinisches Sensorsystem nach Anspruch 1, **dadurch gekennzeichnet, dass** eine Konzentration von Glucose (38a, 38b) ein Bindungsverhalten eines magnetischen und mit Concanavalin-A (42a, 42b) beschichteten Nanopartikels (28a, 28b) an Dextran (40a, 40b) beeinflusst und damit ein Relaxationsverhalten des Nanopartikels (28a, 28b) ändert.

3. Medizinisches Sensorsystem nach einem der vorhergehenden Ansprüche, **dadurch gekennzeichnet, dass** die Signalverarbeitungseinheit (16a - 16c) *ex vivo* angeordnet ist.

4. Medizinisches Sensorsystem nach einem der vorhergehenden Ansprüche, **dadurch gekennzeichnet, dass** die Signalaufnahmeeinheit (16b) zumindest teilweise von einer biodegradierbaren Schicht (48b) umgeben ist.

5. Medizinisches Sensorsystem nach einem der vorhergehenden Ansprüche, **dadurch gekennzeichnet, dass** die Signalaufnahmeeinheit (16a, 16c) eine vollständige Biodegradierbarkeit aufweist.

6. Medizinisches Sensorsystem nach Anspruch 4 oder 5, **dadurch gekennzeichnet, dass** eine Biodegradierbarkeit thermisch mittels Anlegen eines magnetischen Wechselfelds (24a - 24c) mit einer Frequenz von 300-400 kHz von weniger als 100 kA/m, mindestens aber 10 kA/m über eine Zeitdauer von weniger als 30 Minuten, mindestens aber 10 Minuten stimulierbar ist.

7. Medizinisches Sensorsystem nach einem der vorhergehenden Ansprüche, **dadurch gekennzeichnet, dass** die Signalaufnahmeeinheit (16a - 16c) zumindest ein Polymer (50a - 50c) aus Monomeren der Gruppe:
- N-Isopropylacrylamid,
- N,N-Diethylacrylamid,
- Methylvinylether,
- N-Vinylcaprolactam,
- Ethylenoxid-co-Propylenoxid,
- Milchsäure,
- Glycolsäure,
- Trimethylcarbonat
- Acrylat,
- Methacrylat,
- Hydroxyethyl-Metacrylat,
- Vinylester
aufweist.

8. Medizinisches Sensorsystem nach einem der vorhergehenden Ansprüche, **dadurch gekennzeichnet, dass** ein funktionalisiertes magnetisches Nanopartikel (28a - 28c) dazu vorgesehen ist, an ein im Körper (14a- 14c) implantiertes Hydrogel (44a - 44c) reversibel zu binden und diese Bindung ein Relaxationsverhalten des Nanopartikels (28a - 28c) im magnetischen Wechselfeld (24a - 24c) bestimmt.

## Claims

1. A medical sensor system (10a-10c) for detecting at least one feature (12a-12c) in at least one animal and/or human body (14a-14c), having at least one passive signal recording unit (16a-16c), which can be implanted in the animal and/or human body (14a-14c), consists of a biocompatible material, is formed as a sensor (36a-36c) for the concentration of the analyte (30a-30c) glucose (38a-38c), and has at least one hydrogel (44a-44c), which is formed by a copolymer (46a-46c) and in which is arranged at least one magnetic nanoparticle (28a-28c) and forms a passive biosensor, and having at least one signal processing unit (18a-18c), which is arranged spatially separately from the signal recording unit (16a-16c) and comprises a transmitter (20a-20c) and a receiver (22a-22c), **characterised in that** the transmitter (20a-20c) is configured to transmit a magnetic alternating field (24a-24c) to act on the signal recording unit (16a-16c), and the magnetic nanoparticle (28a-28c) is configured to reversibly bind, depending on a concentration of an analyte (30a-30c), either to an analogue (32a-32c) of the analyte (30a-30c) or to a receptor (34a-34c) of the analyte (30a-30c) and of the analogue (32a-32c), and this bond determines a relaxation behaviour of the magnetic nanoparticle (28a-28c) in the magnetic alternating field (24a-24c), wherein the analogue (32a-32c) is formed by a dextran (40a-40c) and the receptor (34a-34c) is formed by concanavalin-A (42a-42c), and the analogue (32a-32c) or receptor (34a-34c) are part of the hydrogel or form a coating of the nanoparticle, and the receiver (22a-22c) is configured to receive a response signal (26a-26c) of the signal recording unit (16a-16c), said response signal being formed by a relaxation signal (52a-52c) of at least one magnetic nanoparticle (28a-28c).

2. The medical sensor system according to Claim 1, **characterised in that** a concentration of glucose (38a, 38b) influences a binding behaviour of a magnetic nanoparticle (28a, 28b), which is coated with concanavalin-A (42a, 42b), to dextran (40a, 40b) and thus changes a relaxation behaviour of the nanoparticle (28a, 28b).

3. The medical sensor system according to one of the preceding claims, **characterised in that** the signal processing unit (16a-16c) is arranged *ex vivo.*

4. The medical sensor system according to one of the preceding claims, **characterised in that** the signal recording unit (16b) is surrounded at least in part by a biodegradable layer (48b).

5. The medical sensor system according to one of the preceding claims, **characterised in that** the signal recording unit (16a, 16c) is fully biodegradable.

6. The medical sensor system according to Claim 4 or 5, **characterised in that** a biodegradability can be stimulated thermally by applying a magnetic alternating field (24a-24c) with a frequency of 300-400 kHz of less than 100 kA/m, but at least 10 kA/m over a period of time of less than 30 minutes, but at least 10 minutes.

7. The medical sensor system according to one of the preceding claims, **characterised in that** the signal recording unit (16a-16c) comprises at least one polymer (50a-50c) from monomers of the following group:
- N-isopropylacrylamide,
- N-N-diethylacrylamide,
- methyl vinyl ether,
- N-vinylcaprolactam,
- ethylene oxide-co-propylene oxide,
- lactic acid,
- glycolic acid,
- trimethylcarbonate,
- acrylate,
- methacrylate,
- hydroxyethyl methacrylate,
- vinyl ester.

8. The medical sensor system according to one of the preceding claims, **characterised in that** a functionalised magnetic nanoparticle (28a-28c) is provided to reversibly bind to a hydrogel (44a-44c) implanted in the body (14a-14c) and this bond determines a relaxation behaviour of the nanoparticle (28a-28c) in the magnetic alternating field (24a-24c).

## Revendications

1. Système de capteurs médicaux (10a - 10c) pour la détection d'au moins une caractéristique (12a - 12c) dans au moins un corps animal et/ou humain (14a - 14c), avec au moins une unité de réception de signaux passive (16a - 16c) implantable dans le corps animal et/ou humain (14a - 14c), constituée d'un matériau biocompatible, conçue comme un capteur (36a - 36c) pour la concentration de l'analyte (30a - 30c) glucose (38a - 38c) et comportant au moins un hydrogel (44a - 44c) constitué d'un copolymère (46a - 46c), dans lequel est agencée au moins une nanoparticule magnétique (28a - 28c) et lequel forme un biocapteur passif, et avec au moins une unité de traitement de signaux (18a - 18c) agencée spatialement à distance de l'unité de réception de signaux (16a - 16c), avec un émetteur (20a - 20c) et un récepteur (22a - 22c), **caractérisé en ce que** l'émetteur (20a - 20c) est conçu pour émettre un champ magnétique alternatif (24a - 24c) pour agir sur l'unité de réception de signaux (16a - 16c), et **en ce que** la nanoparticule magnétique (28a - 28c) est conçue pour se lier de façon réversible soit à un analogon (32a - 32c) d'un analyte (30a - 30c), soit à un récepteur (34a - 34c) de l'analyte (30a - 30c) et de l'analogon (32a - 32c), en fonction d'une concentration de l'analyte (30a - 30c), cette liaison déterminant un comportement de relaxation de la nanoparticule magnétique (28a - 28c) dans le champ magnétique alternatif (24a - 24c), l'analogon (32a - 32c) étant constitué d'un dextrane (40a - 40c) et le récepteur (34a - 34c) est constitué de Concanavaline A (42a - 42c), et l'analogon (32a - 32c) ou le récepteur (34a - 34c) faisant partie de l'hydrogel ou formant un revêtement de la nanoparticule, et **en ce que** le récepteur (22a - 22c) est conçu pour recevoir un signal de réponse (26a - 26c) de l'unité de réception de signaux (16a - 16c), lequel est formé par un signal de relaxation (52a -52c) d'au moins une nanoparticule magnétique (28a - 28c).

2. Système de capteurs médicaux selon la revendication 1, **caractérisé en ce qu'**une concentration de glucose (38a, 38b) influence un comportement de liaison d'une nanoparticule magnétique (28a - 28b) recouverte de Concanavaline A (42a, 42b) avec le dextrane (40a, 40b) et modifie ainsi un comportement de relaxation de la nanoparticule (28a - 28b).

3. Système de capteurs médicaux selon l'une des revendications précédentes, **caractérisé en ce que** l'unité de traitement de signaux (16a - 16c) est agencée *ex vivo.*

4. Système de capteurs médicaux selon l'une des revendications précédentes, **caractérisé en ce que** l'unité de réception de signaux (16b) est au moins partiellement entourée d'une couche biodégradable (48b).

5. Système de capteurs médicaux selon l'une des revendications précédentes, **caractérisé en ce que** l'unité de réception de signaux (16a, 16c) présente une biodégradabilité complète.

6. Système de capteurs médicaux selon la revendication 4 ou 5, **caractérisé en ce que** la biodégradabilité peut être stimulée thermiquement, en appliquant un champ magnétique alternatif (24a - 24c) avec une fréquence de 300-400 kHz inférieure à 100 kA/m, mais d'au moins 10 kA/m, sur une durée inférieure à 30 minutes, mais d'au moins 10 minutes.

7. Système de capteurs médicaux selon l'une des revendications précédentes, **caractérisé en ce que** l'unité de réception de signaux (16a - 16c) comporte au moins un polymère (50a -50c) constitué de monomères du groupe :
- N-isopropylacrylamide,
- N,N-diéthylacrylamide,
- méthylvinyléther,
- N-Vinylcaprolactam,
- oxyde d' éthylène-co-oxyde de propylène,
- acide lactique,
- acide glycolique,
- carbonate de triméthylène,
- acrylique,
- méthacrylique,
- hydroxyéthyl-métacrylique,
- vinylester.

8. Système de capteurs médicaux selon l'une des revendications précédentes, **caractérisé en ce qu'**une nanoparticule magnétique (28a - 28c) fonctionnalisée est prévue pour se lier de façon réversible à un hydrogel (44a - 44c) implanté dans le corps (14a - 14c), et **en ce que** cette liaison détermine un comportement de relaxation de la nanoparticule (28a - 28c) dans le champ magnétique alternatif (24a - 24c).
